# EUROPEAN PATENT SPECIFICATION

(11) **EP 2 207 533 B1**
(45) Date of publication and mention of the grant of the patent: **06.01.2021**
(21) Application number: 08838514.1
(22) Date of filing: 07.10.2008
(51) Int. Cl.: A61K 9/16, A61K 9/20, A61P 29/00, A61K 31/167, A61K 31/192

(54) **DIRECTLY COMPRESSIBLE HIGH FUNCTIONALITY GRANULAR MICROCRYSTALLINE CELLULOSE BASED EXCIPIENT, MANUFACTURING PROCESS AND USE THEREOF**
DIREKT KOMPRIMIERBARER GRANULARER UND MIKROKRISTALLINER HILFSSTOFF AUF ZELLULOSEBASIS MIT HOHER FUNKTIONALITÄT, VERFAHREN ZU SEINER HERSTELLUNG UND SEINE VERWENDUNG
EXCIPIENT À BASE DE CELLULOSE MICROCRISTALLINE GRANULAIRE À FONCTIONNALITÉ ÉLEVÉE DIRECTEMENT COMPRESSIBLE, SON PROCÉDÉ DE FABRICATION ET SON UTILISATION

(30) Priority: 10.10.2007 US 978866 P
(43) Date of publication of application: 21.07.2010
(73) Proprietor: Avantor Performance Materials, LLC, Center Valley, PA 18034 (US)
(72) Inventor: DEORKAR, Nandu, Cedar Knolls New Jersey 07927 (US); FARINA, James, Nazareth Pennsylvania 18064 (US); MIINEA, Liliana, Whitehall Pennsylvania 18052 (US); RANDIVE, Sameer, Thane-west 400 601 (IN)
(74) Representative: EP&C
(86) International application number: PCT/US2008/011555
(87) International publication number: WO 2009/048557

(56) References cited:
- WO-A-2007/049868
- WO-A-2007/052289
- WO-A-2007/134870
- WO-A-2008/020990
- WO-A2-01/89484
- US-A- 5 840 769
- US-A1- 2005 266 075

## Description

### Background of Invention

The most commonly employed means to deliver drug substances is the tablet, typically obtained through the compression of appropriately formulated excipient powders. Tablets should be free of defects, have the strength to withstand mechanical shocks, and have the chemical and physical stability to maintain physical attributes over time and during storage. Undesirable changes in either chemical or physical stability can result in unacceptable changes in the bioavailability of the drug substance. In addition, tablets must be able to release the drug substance in a predictable and reproducible manner. The present invention relates to a novel excipient for use in the manufacture of pharmaceutical solid dosage forms such as tablets. The novel excipient is advantageously combined with at least one drug substance, hereinafter active pharmaceutical ingredient (API), and formed into tablets using a direct compression manufacturing method.

In order to successfully form tablets, the tableting mixture must flow freely from a feeder hopper into a tablet die, and be suitably compressible. Since most APIs have poor flowability and compressibility, APIs are typically mixed with varying proportions of various excipients to impart desired flow and compressibility properties. In typical practice, a compressible mixture is obtained by blending an API with excipients such as diluents/fillers, binders/adhesives, disintegrants, glidants/flow promoters, colors, and flavors. These materials may be simply blended, or may be wet or dry granulated by conventional methods. Once mixing is complete, a lubricating excipient is typically added and the resulting material compressed into tablets.

Unfortunately, there are few general rules regarding excipient compactibility with particular APIs. Therefore, when developing tablet formulations to meet particular desired characteristics, pharmaceutical scientists typically must conduct an extensive series of experiments designed to determine which excipients are physically and chemically compatible with a specific API. Upon completion of this work, the scientist deduces suitable components for use in one or more trial compositions.

Two conventional methods of making tablets are dry blending followed by direct compression, and granulation followed by direct compression. In a typical direct compression process, the API is blended with the desired excipients such as diluent/filler, binder, disintegrant, glidant, and colors. Once blending is complete a lubricating excipient is added and the resulting material is compressed into tablets.

The direct compression method is limited by and dependent on the specific API properties, and further upon the combination of the various excipients. Therefore granulation of the excipients with the API is typically employed in order to achieve satisfactory tablets and/or improve tablet production speed. Traditional methods of granulation include dry granulation, wet granulation, and spray granulation. Each of these methods has limitations regarding the particles produced from the process.

The dry granulation method consists of mixing the components to form a blend which is roll compacted. This process is limited as the particles are not held together strongly and easily fall apart. Roll compaction processing also results in reduction of compactibility of many excipients.

Wet granulation is a process in which excipients are bound together in the presence of a liquid binder in a blender system, to produce a wet granular blend which is dried. Spray granulation is a process in which excipients are bound together in a fluidized bed. These processes are batch processes, which limits production speed, and can produce a variable product.

These conventional processes are utilized to produce particles with improved powder flow characteristics to produce tablets having improved physical characteristics. However, these processes are time consuming and may not be compatible with many APIs.

Various attempts have been made to produce improved excipients. U.S. Patent No. 4,675,188 to Chu et al. discloses a granular directly compressible anhydrous dicalcium phosphate excipient which purports to have a particle size sufficient for efficient direct compression tableting. According to the disclosure, dicalcium phosphate is dehydrated, and then granulated with a binder. The resulting product is purportedly a granular anhydrous dicalcium phosphate, characterized in that at least 90 percent of the particles are larger than 44 microns. This granular product purports to improve over commonly used precipitated anhydrous dicalcium phosphate, which is a fine, dense powder that must be agglomerated with a binder such as starch before it can be used in direct compression tableting. The process disclosed in this patent consists of coating anhydrous calcium phosphate with starch or another binder, purportedly resulting in binding of calcium phosphate particles to each other forming large particles. However, this granulated product is not a universal excipient, in that it lacks other necessary excipients, such as disintegrants, that are necessary to produce a pharmaceutically acceptable tablet after compression.

U.S. Patent No. 6,746,693 discloses an agglomerated microcrystalline cellulose blend containing silicon dioxide, purported to have improved compressibility. The disclosure states that silicon dioxide is a critical component to improve compressibility. The two step process described includes spray granulation followed by wet granulation, and does not provide a complete universal excipient.

A commercially available excipient, Ludipress®, is disclosed in EP 0192173B1. Ludipress® is composed of lactose, crospovidone, and povidone. Lactose is known to have better flowability than microcrystalline cellulose due to inherently different particle shape and morphology. Lactose and povidone are water soluble components that mix well with a third non-water soluble component for granulation by spray drying. There is no disclosure of a complete universal excipient including two or more insoluble components, or a specific particle morphology to enable increased flowability, compactibility with various APIs and varying degrees of loading.

There exists therefore a need in the pharmaceutical industry for a complete and universal directly compressible high functionality granular excipient that consists of not only filler but also a binder and a disintegrant. The desired excipient is also compatible with a wide range of APIs, and has a particle shape, size, and morphology to provide optimal flowability and compressibility. This high functionality excipient would simplify tableting and may require one step mixing of the API and lubricant before direct compression.
US 5 840 769 , WO 2007/052289, WO 2007/049868 disclose tablets comprising at least one active pharmaceutical ingredient and granules including: a) microcrystalline cellulose; b) at least one binder; and c) at least one disintegrant
WO 2008/020990 and WO 01/89484 are earlier applications, but later published and disclose tablets comprising at least one active pharmaceutical ingredient and granules including: a) microcrystalline cellulose; b) at least one binder; and c) at least one disintegrant.

### Summary of the invention

An illustrative aspect of the present invention is a composition comprising substantially homogeneous particles including:
about 75% to about 98% microcrystalline cellulose;
about 1% to about 10% at least one binder; and
about 1% to about 15% at least one disintegrant,
wherein the microcrystalline cellulose, binder and disintegrant are indistinguishable when viewed with a SEM, thereby forming substantially homogeneous, spherical particles wherein the binder is hydroxypropyl methylcellulose and the disintegrant is cross-linked polyvinylpyrrolidone,
wherein the composition is formed by spraying aqueous slurry comprised of the microcrystalline cellulose, binder and disintegrant.

Another illustrative aspect of the present disclosure is an excipient comprising about 75% to about 98% microcrystalline cellulose, about 1% to about 10% at least one binder, and about 1% to about 15% at least one disintegrant, wherein the excipient is formed by spraying an aqueous slurry comprised of the microcrystalline cellulose, binder and disintegrant.

Yet another illustrative aspect of the present invention is a method of making an excipient. The method comprises mixing a binder in water to from a viscous solution; homogenizing microcrystalline cellulose , and a disintegrant into the viscous solution to form a slurry; and spray dry granulating the slurry to form substantially homogeneous particles of excipient wherein the binder is hydroxypropyl methylcellulose and the disintegrant is cross-linked polyvinylpyrrolidone and wherein the excipient comprises 75% to 98% microcrystalline cellulose; 1% to 10% said at least one binder; and 1% to 15% said at least one disintegrant.

Still another illustrative aspect of the present invention is another method of making an excipient. The method comprises dissolving hydroxypropyl methylcellulose in water to form a viscous solution; mixingmicrocrystalline cellulose and cross-linked polyvinylpyrrolidone into the viscous solution to form a slurry; spraying the slurry to form substantially homogeneous particles.

A further illustrative aspect of the present invention is a pharmaceutical tablet comprising at least one active pharmaceutical ingredient; and an excipient of substantially homogeneous particles including:
a) microcrystalline cellulose;
b) at least one binder; and
c) at least one disintegrant,
   wherein the binder is hydroxypropyl methylcellulose and the disintegrant is cross-linked polyvinylpyrrolidone, wherein the excipient is formed by spraying an aqueous slurry comprised of the microcrystalline cellulose, binder and disintegrant and
   wherein the excipient includes:
   75% to 98% microcrystalline cellulose;
   1% to 10% said at least one binder; and
   1% to 15% said at least one disintegrant..

Yet a further illustrative aspect of the present invention is a method of making a pharmaceutical tablet. The method comprises mixing at least one active pharmaceutical ingredient and an excipient according to the invention and compressing the resulting mixture to form a tablet. The excipient comprises substantially homogeneous particles including microcrystalline cellulose, at least one binder, and at least one disintegrant.

### Brief Description of Drawings

Figure 1 is an illustration of SEM micrographs of the improved excipient of the present invention produced according to Example 1.
Figure 2 is an illustration of SEM micrographs of the improved excipient of the present invention produced according to Example 2.
Figure 3 is an illustration of SEM micrographs of microcrystalline cellulose.
Figure 4 is an illustration of SEM micrographs of a commercially available excipient, Prosolv®90.
Figure 5 is an illustration of SEM micrographs of a commercially available excipient, Ludipress®
Figure 6 is an illustration of SEM micrographs of an excipient manufactured by conventional high shear wet granulation method according to Example 4.
Figure 7 is an illustration of a flowability index comparison of an excipient made by conventional high shear wet granulation according to example 4 and the improved excipient of the present invention produced according to Examples 1, 2 and 3.
Figure 8 is an illustration of SEM micrographs of multiple samplings of the improved excipient of the present invention produced according to Example 3.

### Detailed Description

There is provided an improved excipient comprising substantially homogeneous particles of a compressible, high functionality granular microcrystalline cellulose based excipient. The improved excipient provides enhanced flowability/good flow properties, excellent/high compactibility, and increased API loading and blendability as compared to the individual components, and as compared to conventional excipients formed from the same materials.

The improved excipient has strong intraparticle bonding bridges between the components, resulting in a unique structural morphology including significant open structures or hollow pores. The presence of these pores provides a surface roughness that is the ideal environment for improved blending with an API. Excellent blendability is an essential characteristic of an excipient as it allows tablets to be produced that contain a uniform amount of the API. Additionally, this improved excipient includes the necessary excipients, except for the optional lubricant, that are required to produce a pharmaceutically acceptable tablet.

The improved excipient is engineered to have particle size that results in the excipient being directly compressible, complete, and universal excipient for making pharmaceutical tablets. The excipient is considered complete since it includes a diluent, a binder and a disintegrant, and universal since it is surprisingly compatible with a variety of APIs. The components and physical characteristics of the improved excipient were carefully chosen and optimized to ensure its use in formulating a wide range of APIs.

The universality of this excipient overcomes the need for the traditional time consuming approach to formulation development, wherein the scientist develops a custom blend of various excipients to optimize flowability and compressibility for the particular API. It was unexpectedly discovered that the disclosed composition and process of making the improved excipient provides a substantially homogeneous, strong spherical particle having high increased porosity that provides good flowability and high compactibility. The improved excipient typically has an aerated bulk density of about 0.1-0.4 g/cc.

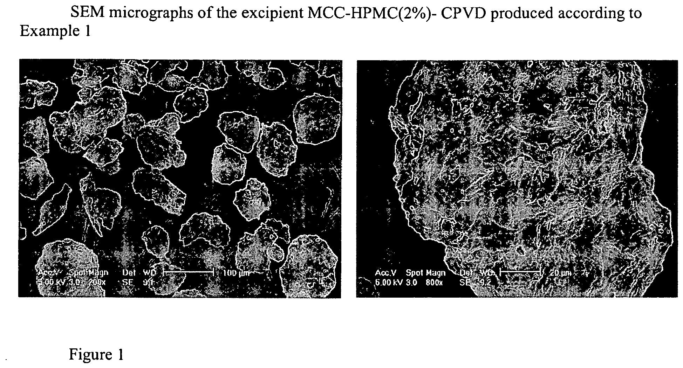

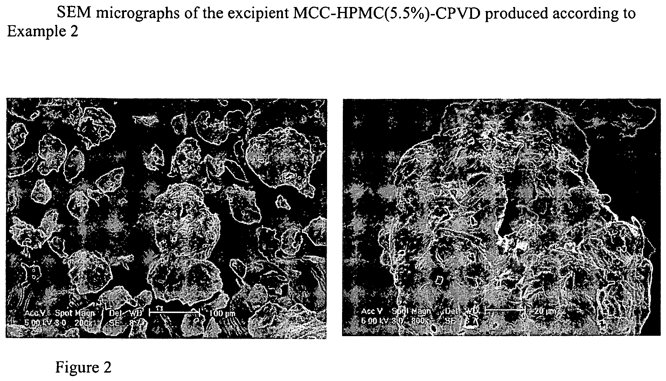

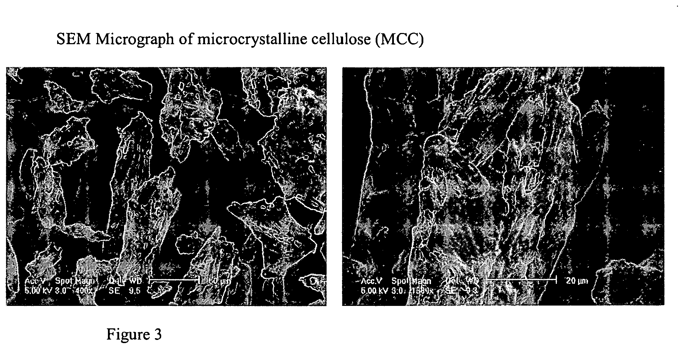

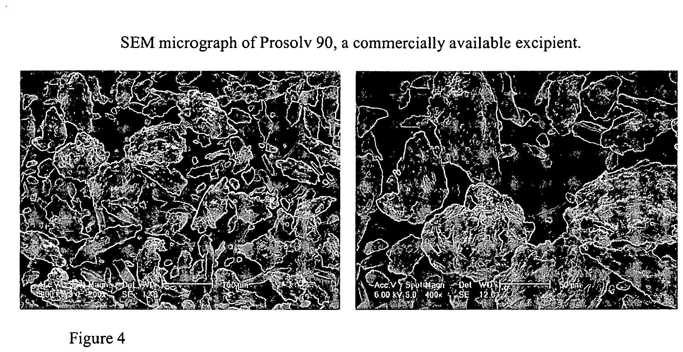

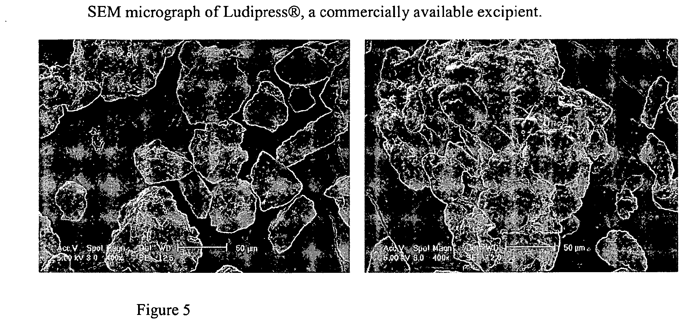

Unprocessed microcrystalline cellulose (MCC) has a needle-like shape when viewed under SEM (as illustrated in Figure 3). The particle morphology of the improved excipient disclosed herein is unexpectedly unique as a substantially homogeneous spherical structure with holes or pores and hollow portions in the particles that can improve API loading capacity. As is illustrated in Figures 1 and 2, the term substantially homogeneous is meant herein to denote a structure in which the individual components cannot be distinguished under SEM scan. This contrasts with traditional excipients such as Prosolv® (as illustrated in Figure 4) and Ludipress® (as illustrated in Figure 5). These conventionally produced excipients do not produce the substantially homogeneous particle morphology of the improved excipient, but instead are composed of easily distinguished, agglomerated particles bonded together with either fiber like filaments. The granules formed in the traditional and other disclosed processes are seen as a simple bonding of particles into irregularly shaped granules produced by agglomeration of distinct particles. It is common for these agglomerated particles to separate into the distinct components during transport or rough handling. The continuous spherical particles of the improved excipient, while including hollow portions, are unexpectedly robust and are not friable during handling and processing.

In the present invention, MCC is processed in combination with a polymeric binder and a cross-linked hygroscopic polymer to produce spherical particles having high porosity and strong intraparticle binding. The polymeric binder is selected from the class of cellulosic polymers or organic synthetic polymers having thermal stability at about 80 °C to about 120 °C, dynamic viscosity in the range of about 2 mPa to about 50 mPa for a water solution of about 0.5% to about 5% wt/vol, water solubility in the range of about 0.5% to about 5% wt/vol and providing a surface tension in the range of about 40 dynes/cm to about 65 dynes/cm for about 0.5% to about 5% wt/vol water solution. Preferred binders from this class include hydroxypropyl methylcellulose, hydroxyethyl cellulose, hydroxypropyl cellulose, sodium carboxymethyl cellulose, and polyvinyl alcohol-polyethylene glycol graft copolymer and vinylpyrrolidone-vinyl acetate copolymer. Presently preferred is hydroxypropyl methylcellulose (HPMC). The cross-linked hygroscopic polymer disintegrant is preferably crospovidone (CPVD). As is seen in Figures 1 and 2, the processed particles are a substantially homogeneous composition of spheres with porous portions leading to at least partially hollow portions of the spheres. The granules are produced by the actual physical binding of the slurry mixture that becomes distinct particles when blown out of the nozzle. The porosity and hollow portions result in improved API loading and blendability.

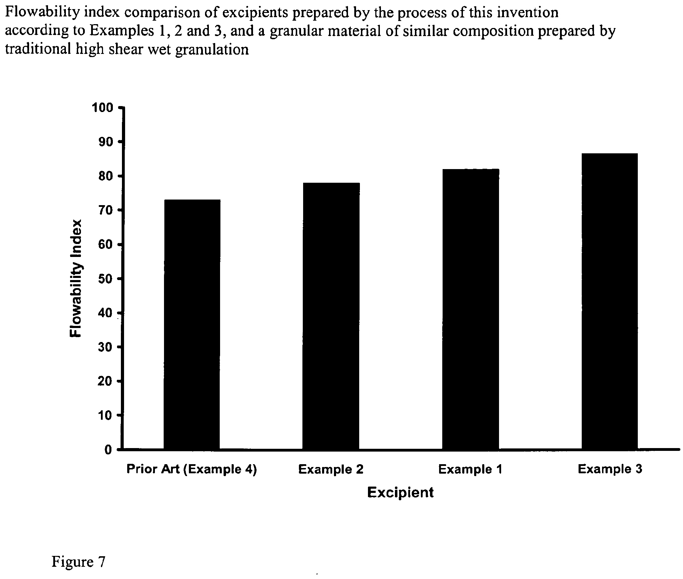

The improved excipient has excellent flowability. In general, when particle flow is poor, additional glidants such as silicon dioxide are added to improve flow. If the powder flow is not sufficient, poor tablet productivity will result. Characterization of the improved excipient particles by the Carr method, well know in the art, showed a flowability index that exceeds 80, where a flowability index over 70 indicates good flowability. As is seen in Example 6, a Hosokawa powder tester, a test instrument that measures powder characteristics using a set of automated tests using the Carr method was used to determine that the improved excipient of Example 1 has a flowability index of 82. Fig. 7 illustrates a comparison of flowability index for a conventionally prepared excipient according to Example 4 with the improved excipient of the present invention.

As illustrated in Example 5, the granules of the material produced according to the invention are stronger than those of a similar material produced by a traditional high shear wet granulation process.

As illustrated in Examples 13 and 15, the improved excipient of the present invention produced acceptable tablets by direct compression when directly mixed with as low as about 5% API or as high as about 50% API. This indicates universal application and use of the material produced according to this invention.

The process disclosed herein is a novel form of the spray drying granulation process. The new process consists of the homogenization of all three components of the excipient in the presence of water to create a slurry of the components. The homogenization process is carried out to bring the two insoluble components, MCC and a disintegrant, in contact with each other and bound in close association with a viscous binder solution, for example hydroxypropyl methylcellulose. The evaporation of water at a high rate at high temperatures of 120 °C or more and the local action of HPMC holding all components together produces particle with unique shape and morphology.

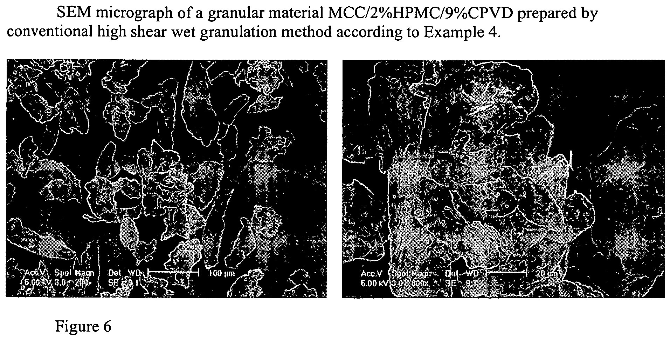

In contrast, a traditional spray drying method uses compositions of one or two soluble components. Example 4, Figure 6 illustrates the composition components of the present invention processed by the traditional wet granulation method. The material produced from the conventional high shear wet granulation process consisted of needle like friable particles that did not perform as well as the product formed by the present method, as illustrated in Examples 1 and 3. Compressiblity decreased, resulting in a 1.8 times decrease in the hardness of the placebo tablets pressed from the conventionally produced material as compared to the improved according to Example 1, see Example 7. The particle morphology is composed of irregular particles bonded together by simple intergranular bridges, as seen in Figure 6.

The components of the improved excipient are processed by an improved wet homogenization/spray dry granulation method. In this process, a slurry is formed of two water insoluble components (typically with a large difference in composition between the two water insoluble components) and a third water soluble component. The resulting slurry is granulated to a desired particle size, typically greater than about 50 µm, preferably about 50 µm to about 250 µm, and more preferably about 90µm to about 150 µm.

The excipient is formed by processing, or homogenizing, MCC with the polymeric binder and a cross-linked hygroscopic polymer disintegrant. In an illustrative embodiment, the excipient is formed from about 75% to about 98% MCC, in combination with about 1% to about 10% binder and about 1% to about 15% disintegrant. In a preferred embodiment, the excipient is formed from about 80% to about 90% MCC, about 2% to about 8% binder and about 3% to about 12% disintegrant. In a more preferred embodiment, the excipient is formed from about 85% to about 93%, about 2% to about 5% binder and about 10% disintegrant.

It has further been determined that varying the ratio of MCC and disintegrant to the binder affects the density of the final excipient. In an illustrative example, utilizing HPMC as the binder 5.5% HPMC yields an excipient with an aerated bulk density of 0.2 g/cc, see Example 2, wherein 2% HPMC yields an excipient with an aerated bulk density of 0.3 g/cc, see Example 1. The increase in bulk density indicates a lower porosity.

The use of the improved excipient will reduce formulation development to a series of blending steps: blending of an API with the improved excipient (which contains the essential components of tablet formulation, diluent, binder and disintegrant) and optionally a lubricant. Suitable APIs are limited only in that they are compatible with MCC and the other excipient components. The blending process will be followed by pressing high quality tablets by direct compression.

Therefore, the composition and processing steps disclosed herein produce an improved excipient exhibiting novel final particle morphology and unexpectedly improved compressibility.

### Example 1: Preparation of microcrystalline cellulose- 2% hydroxypropyl methylcellulose - crospovidone excipient according to the present invention.

The improved excipient consists of microcrystalline cellulose at 85%, hydroxypropyl methyl cellulose at 2%, and crospovidone at 13%. The excipient was produced by a wet homogenization/spray dry granulation process. The apparatus used for the production of the excipient was a Co-current atomizer disc type with the disc RPM between 12000 and 25000 and the inlet temperatures of 180-250 °C. Powdered MCC was converted into slurry in a mixing chamber with deionized water to give a concentration of 23.34%. The other components, HPMC and crospovidone were also converted to a slurry with deionized water in a separate mixing chamber at 60 °C to a concentration of 5.93%. The HPMC/crospovidone mixture was then transferred into the chamber containing the MCC slurry and homogenized into a uniform mixture at 40-60 °C for 2 hours using circulating shear pump and an agitator to keep solid suspended in the solution to form a uniform slurry. The slurry mixture was then spray dried through a rotary nozzle at a motor frequency of 33 Hz in the presence of hot air at an outlet temperature of 106-109 °C. This constitutes the granule formation step. The fines were removed in a cyclone and the final product was collected to give the new improved excipient. SEM micrographs of the excipient of Example 1 are seen in Figure 1. Unless otherwise noted, all SEM micrographs herein were recorded using a FEI XL30 ESEM (environmental scanning electron microscope), voltage 5 kV, spot size 3, SE detector. The samples were sputtered with Iridium before SEM analysis (sputtering time 40 sec.)

The compressibility, aerated bulk density and tapped bulk density of the granular material were measured using a Powder Tester (Hosokawa Micron Corporation) Model PT-S. A computer which uses the Hosokawa Powder Tester software was used to control the Hosokawa Powder Tester during the measurement operation, enabling simple use and data processing. For measuring the aerated bulk density and tapped bulk density a 50 cc cup was employed. The standard tapping counts for measuring the tapped bulk density were 180 and the tapping stroke was 18 mm. D50 value was calculated based on the data collected in a "particle size distribution" measurement. An Air Jet Sieving instrument (Hosokawa Micron System) was used to determine the particle size distribution of the granular material. A set of four sieves (270 mesh, 200 mesh, 100 mesh and 60 mesh) was used. The sieving time for each sieve was 60 sec, while the vacuum pressure was maintained at 12-14 in. H₂O. The sample size was 5 g.

The "loss on drying" (LOD) value was determined using a Mettler Toledo Infrared Dryer LP16. The set temperature was 120 °C and the analysis was stopped when constant weight was reached.

**Table 1**

| Powder Characteristics | Value |
|---|---|
| 1. Compressibility | 16.1% |
| 2. D50 | 113 um |
| 3. Aerated bulk density | 0.29 g/cc |
| 4. Tapped bulk density | 0.35 g/cc |
| 5. LOD | 3.0 % |

### Example 2: Preparation of microcrystalline cellulose- 5.5% hydroxypropyl methylcellulose - crospovidone excipient according to the present invention.

The excipient consists of microcrystalline cellulose at 85.5%, hydroxypropyl methyl cellulose at 5.5%, and crospovidone at 9%. The excipient was produced by a wet homogenization/spray drying granulation process. The apparatus used for the production of the excipient is a Co-current atomizer disc type with the disc RPM between 12000 - 25000 and the inlet temperatures of 180 - 250 °C. After granulation a cyclone separation device was used to remove the fines. Powdered MCC was converted into a slurry using deionized water in a mixing chamber to reach a concentration of 25.12%. The other components HPMC and crospovidone were first dry mixed and then also converted into a slurry with deionized water in a separate mixing chamber to a concentration of 11.4%. The HPMC/crospovidone mixture was then transferred into the chamber containing the MCC slurry and homogenized into a uniform mixture at 40-60 °C for 2 hours using circulating shear pump and a agitator to keep solid suspended in the solution to form uniform slurry The slurry mixture was then spray dried through a rotary nozzle at the motor frequency of 40.1 Hz in the presence of hot air at an outlet temperature of 106-109 °C. This constitutes the granule formation step. The fines were removed in a cyclone and the final product was collected, see Figure 2.

The powder characteristics were determined as described in example 1.

**Table 2**

| Powder Characteristics | Value |
|---|---|
| 1. Compressibility | 19.7% |
| 2. D50 | 104 um |
| 3. Aerated bulk density | 0.20 cc/g |
| 4. Tapped bulk density | 0.25 cc/g |
| 5. LOD | 2.0 % |

### Example 3

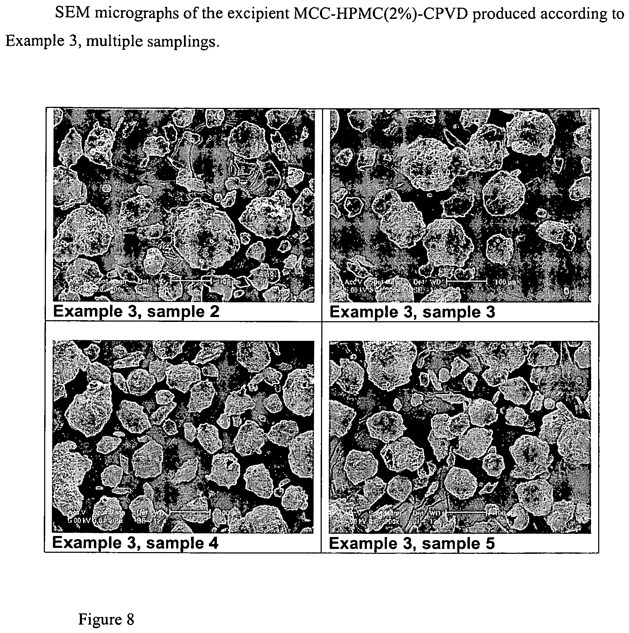

The excipient consists of microcrystalline cellulose at 89%, hydroxypropyl methyl cellulose at 2%, and crospovidone at 9%. The excipient was produced by a wet homogenization/spray drying granulation process. The apparatus used for the production of the excipient was a Co-current atomizer disc type with the disc RPM between 12000 - 25000 and the inlet temperatures of 180 - 250 °C. After granulation a cyclone separation device was used to remove the fines. The production of the granular excipient begins with converting powdered MCC (which consists of rod like particles) into a slurry using deionized water in a mixing chamber to a concentration of 23.34%. The other components HPMC and crospovidone were first dry mixed and also converted to a slurry with deionized water in a separate mixing chamber to a concentration of 10.1%. The HPMC/crospovidone mixture was then transferred into the chamber containing the MCC slurry and homogenized into a uniform mixture at 40-60 °C for 2 hours using a circulating shear pump and a agitator to keep solid suspend in the solution to form uniform slurry. The slurry mixture was then spray dried through a rotary nozzle at the motor frequency of 32.5 Hz in the presence of hot air at an outlet temperature of 106-109 °C. This constitutes the granule formation step. The fines were removed in a cyclone and the final product was collected. The uniformity of product taken from several samplings is illustrated in Figure 8.

The powder characteristics were determined as described in example 1.

**Table 3**

| Powder Characteristics | Value | |
|---|---|---|
| 1. | Compressibility | 16.5 % |
| 2. | D50 | 117 um |
| 3. | Aerated bulk density | 0.27 g/cc |
| 4. | Tapped bulk density | 0.34 g/cc |
| 5. | LOD | 5.7 % |

### Example 4: High Shear Wet Granulation of Microcrystalline Cellulose (89%)-HPMC (2%)-Crospovidone (9%)

133.5 g microcrystalline cellulose, 3.0 g Hydroxypropyl methylcellulose and 13.5 g crospovidone was placed in a 1 L stainless steel bowl. The bowl was attached to a GMX.01 vector micro high shear mixer/granulator (Vector Corporation). The dry mixture was mixed for 2 minutes at 870 rpm impeller speed and 1000 rpm chopper speed. 70 g of deionized water ("the liquid binder") was added to the dry blend, drop by drop, using a peristaltic pump at a dose rate of 16 rpm. During the liquid binder addition the impeller speed was 700 rpm and the chopper speed was 1500 rpm. The wet massing time was 60 seconds maintaining the same impeller and chopper speed as during the liquid addition. Following the granulation, the wet granular material was dried in a tray at 60 °C. The resulted granular material (moisture content 2.35%) was screened through a 30 mesh sieve. The yield of the granular material that passed through 30 mesh screen was 116.73 g (79.3% referenced to dry starting materials and dry product). See Figure 6.

### Example 5: Granules friability test for the Example 1 excipient and the material obtained by high shear wet granulation as per Example 4.

75 - 100 g of granular material were loaded in a 4 L V-Blender and tumbled for 2 h. The granular material was collected and analyzed. An Air Jet Sieving instrument (Hosokawa Micron System) was used to determine the particle size distribution of the granular material before and after tumbling. A set of four sieves (270 mesh, 200 mesh, 100 mesh and 60 mesh) was used. The sieving time for each sieve was 60 sec, while the vacuum pressure was maintained at 12-14 in. H₂O. The sample size was 5 g.

**Table 4**

| Sample | % Particles with diameter less than 50 microns before tumbling | % Particles with diameter less than 50 microns after tumbling |
|---|---|---|
| Example 4 | 14 | 30 |
| Example 1 | 5 | 4 |

### Example 6. Comparison of Powder Characteristics for Example 1 and Example 3 excipient and the material obtained by high shear wet granulation as per example 4.

The powder characteristics of the granular materials were measured using a Powder Tester (Hosokawa Micron Corporation) Model PT-S. The Hosokawa Powder tester determines flowability of dry solids in accordance with the proven method of R. L. Carr. A computer which uses the Hosokawa Powder Tester software was used to control the Hosokawa Powder Tester during the measurement operation, enabling simple use and data processing. For measuring the aerated bulk density and tapped bulk density a 50 cc cup was employed. The standard tapping counts for measuring the tapped bulk density were 180 and the tapping stroke was 18 mm.

**Table 5**

| Property | Example 3 | | Example 4 | | Example 1 | |
|---|---|---|---|---|---|---|
| | Value | Index | Value | Index | Value | Index |
| Angle of repose (deg) | 30.9 | 22.0 | 37.9 | 18.0 | 34.9 | 20.0 |
| Aerated Bulk Density (g/cc) | 0.272 | | 0.299 | | 0.296 | |
| Packed Bulk Density (g/cc) | 0.339 | | 0.389 | | 0.353 | |
| Compressibility | 19.8 | 17.5 | 23.1 | 16.0 | 16.1% | 19.5 |
| Angle of Spatula Before Impact | 31.6 | | 60.1 | | 44.6 | |
| Angle of Spatula After Impact | 23.4 | | 42.5 | | 32.8 | |
| Angle of spatula (avg) | 27.5 | 24.0 | 51.3 | 16.0 | 38.7 | 19.5 |
| Uniformity | 2.9 | 23.0 | 2.9 | 23.0 | 2.1 | 23.0 |
| Total Flowability Index | | 86.5 | | 73.0 | | 82.0 |

### Example 7: Comparison of hardness vs. compression force profiles for placebo tablets prepared using Example 1 excipient and the material obtained by high shear wet granulation as per example 4.

Approximately 0.5 g tablets were pressed from the corresponding granular material at various compression forces using a Carver manual press and a 13 mm die. The dwell time was 5 seconds. No lubricant was added. The hardness of the tablets was measured using a Varian, Benchsaver™ Series, VK 200 Tablet Hardness Tester. The values recorded in the table below are an average of three measurements.

**Table 6**

| Compression force (pound-force) | Hardness (kp) | |
|---|---|---|
| | Example 4 | Example 1 |
| 3000 | 18.43 | 31.0 |
| 2000 | 12.93 | 22.2 |
| 1000 | 5.7 | 10.1 |

Example 8: Comparison of Hausner Ratio and Carr's Compressibility Index (%) of microcrystalline cellulose from different commercial sources, commercial co-processed excipients containing microcrystalline cellulose, and Example 1, 2 and 3 excipients.

Using the aerated and tapped bulk density, Carr's compressibility index and Hausner ratio can be calculated. A value of 20-21% or less for the Carr's compressibility index and a value below 1.25 for the Hausner ratio indicate a material with good flowability.

**Table 7**

| Excipient Brand Name | Hausner Ratio | Compressibility Index (%) |
|---|---|---|
| Emcocel 90 | 1.32 | 24.5 |
| Avicel PH 102 | 1.32 | 24.2 |
| Prosolv 90 | 1.23 | 18.9 |
| Example 4 | 1.30 | 23.1 |
| Example 2 | 1.25 | 19.7 |
| Example 1 | 1.19 | 16.1 |
| Example 3 | 1.22 | 16.5 |

| | | |
|---|---|---|
| Emcocel 90, Avicel PH 102 - brands of microcrystalline cellulose Prosolv 90 - silicified microcrystalline cellulose | | |

### Example 9: Disintegration Time vs. Hardness for Placebo Tablets of MCC Based Granular Excipients

Approximately 0.5 g tablets were pressed from the corresponding granular material at a compression force of 3000 Ibs-f using a Carver manual press and a 13 mm die. The dwell time was 5 seconds. No lubricant was added. The disintegration experiments were performed with a Distek Disintegration System 3100, using 900 mL deionoized water at 37 °C.

**Table 8**

| Tablet | Hardness (kp) | Disintegration time (sec) |
|---|---|---|
| Example 1 | 31.0 | 55.5 |
| Example 2 | 30.3 | 150 |
| Example 3 | 26.33 | 42 |

### Example 10 Powder properties of a mixture of 5% Acetaminophen with Example 1 excipient.

7.9 g of Acetaminophen were blended with 150 g of Example 1 excipient in a 4 L V-blender for 1 h 30 min. The powder characteristics were measured using the same method mentioned in Example 6. The D50 value was calculated based on the data collected in a "particle size distribution" measurement similar to the one described in Example 5.

**Table 9**

| Powder Characteristics | Value | |
|---|---|---|
| 1. | Compressibility index | 20.7% |
| 2. | D50 | 116 um |
| 3. | Aerated bulk density | 0.29 g/cc |
| 4. | Tapped bulk density | 0.36 g/cc |

### Example 11: Powder properties of a mixture of 30% Acetaminophen with Example 1 excipient.

64.9 g of Acetaminophen were blended with 150 g of Example 1 excipient in a 4 L V-blender for 1 h 30 min. The powder characteristics were measured using the same method mentioned in Example 6. The D50 value was calculated based on the data collected in a "particle size distribution" measurement similar to the one described in Example 5.

**Table 10**

| Powder Characteristics | Value | |
|---|---|---|
| 1. | Compressibility index | 32.9 % |
| 2. | D50 | 117 um |
| 3. | Aerated bulk density | 0.28 g/cc |
| 4. | Tapped bulk density | 0.42 g/cc |

### Example 12. Powder properties of a mixture of 30% Ibuprofen with Example 1 excipient.

64.3 g of Ibuprofen were blended with 150 g of Example 1 excipient in a 4 LV-blender for 1 h 30 min. The powder characteristics were measured using the same method mentioned in Example 6. The D50 value was calculated based on the data collected in a "particle size distribution" measurement similar to the one described in Example 5.

**Table 11**

| Powder Characteristics | Value | |
|---|---|---|
| 1. | Compressibility index | 27.6 % |
| 2. | D50 | 105 um |
| 3. | Aerated bulk density | 0.28 g/cc |
| 4. | Tapped bulk density | 0.39 g/cc |

### Example 13. Preparation of 5% Acetaminophen tablets using the powder blend prepared according to Example 10.

Approximately 0.5 g tablets were pressed from the corresponding granular material at various compression forces using a Carver manual press and a 13 mm die. The dwell time was 5 seconds. No lubricant was added. The hardness of the tablets was measured using a Varian, Benchsaver™ Series, VK 200 Tablet Hardness Tester. The values recorded in the table below are an average of three measurements. The disintegration experiments were performed with a Distek Disintegration System 3100, using 900 mL deionoized water at 37 °C.

**Table 12**

| Compression Force (pound-force) | Hardness (kp) | Disintegration in Water |
|---|---|---|
| 4000 | 33.2 | 90 sec |
| 3000 | 28.3 | 52 sec |
| 2000 | 21.8 | 15 sec |

### Example 14. Preparation of 30% Acetaminophen tablets using the powder blend prepared according to Example 11.

Approximately 0.5 g tablets were pressed from the corresponding granular material at various compression forces using a Carver manual press and a 13 mm die. The dwell time was 5 seconds. No lubricant was added. The hardness of the tablets was measured using a Varian, Benchsaver™ Series, VK 200 Tablet Hardness Tester. The values recorded in the table below are an average of three measurements. The disintegration experiments were performed with a Distek Disintegration System 3100, using 900 mL deionized water at 37 °C.

**Table 13**

| Compression Force (pound-force) | Hardness (kp) | Disintegration in Water |
|---|---|---|
| 4000 | 17.4 | 18 sec |
| 3000 | 13.0 | 19 sec |
| 2000 | 8.8 | 16 sec |

### Example 15. Preparation of 50% Naproxen Sodium/Example 3

80 g Naproxen Sodium were blended with 80 g example 3 excipient and 800 mg (0.5%) amorphous silica (glidant) in a 4 L V-blender for 1 h 30 min. Approximately 0.5 g tablets were pressed from the corresponding granular material at various compression forces using a Carver manual press and a 13 mm die. The dwell time was 5 seconds. No lubricant was added. The hardness of the tablets was measured using a Varian, Benchsaver™ Series, VK 200 Tablet Hardness Tester. The values recorded in the table below are an average of three measurements. The disintegration experiments were performed with a Distek Disintegration System 3100, using 900 mL deionoized water at 37 degrees Celsius.

**Table 14**

| | |
|---|---|
| Hardness vs. Compression Force Profiles for Tablets obtained from 50% Na Naproxen/Example 3 excipient | |

| Compression Force (lbs-force) | Hardness (kp) |
|---|---|
| 4000 | 16.77 |
| 3000 | 14.3 |
| 2000 | 11.77 |

**Table 15**

| | |
|---|---|
| Disintegration time for Tablets obtained from 50% Naproxen Sodium/Example 3 excipient | |

| Tablet Composition (hardness) | Disintegration time |
|---|---|
| 50% Na Naproxen/Example 3 (16.77 kp) | 11 min |
| 50% Na Naproxen/Example 3 (14.3 kp) | 10 min 20 sec |

Unless otherwise noted, all percentages are weight/weight percentages.

## Claims

1. A composition comprising substantially homogeneous particles including:
about 75% to about 98% microcrystalline cellulose;
about 1% to about 10% at least one binder; and
about 1% to about 15% at least one disintegrant,
wherein the microcrystalline cellulose, binder and disintegrant are indistinguishable when viewed with a SEM, thereby forming substantially homogeneous, spherical particles wherein the binder is hydroxypropyl methylcellulose and the disintegrant is cross-linked polyvinylpyrrolidone,
wherein the composition is formed by spraying aqueous slurry comprised of the microcrystalline cellulose, binder and disintegrant.

2. The composition of claim 1 wherein the composition includes:
about 80% to about 90% microcrystalline cellulose;
about 2% to about 8% at least one binder; and
about 3% to about 12% at least one disintegrant.

3. The composition of Claim 1 wherein the composition includes:
about 85% to about 93% microcrystalline cellulose;
about 2% to about 5% at least one binder; and
about 10% at least one disintegrant.

4. A method of making an excipient comprising:
mixing a binder in water to from a viscous solution; homogenizing microcrystalline cellulose and a disintegrant into the viscous solution to form a slurry; and spray dry granulating the slurry to form substantially homogeneous particles of excipient wherein the binder is hydroxypropyl methylcellulose and the disintegrant is cross-linked polyvinylpyrrolidone and wherein the excipient comprises
75% to 98% microcrystalline cellulose;
1% to 10% said at least one binder; and
1% to 15% said at least one disintegrant.

5. The method of claim 4 comprising:
about 80% to about 90% microcrystalline cellulose;
about 2% to about 8% at least one binder; and
about 3% to about 12% at least one disintegrant.

6. The method of Claim 4 comprising:
about 85% to about 93% microcrystalline cellulose;
about 2% to about 5% at least one binder; and
about 10% at least one disintegrant.

7. A method of making an excipient comprising:
dissolving hydroxypropyl methylcellulose binder in water to form a viscous solution homogenising microcrystalline cellulose and cross-linked polyvinylpyrrolidone disintegrant
Into the viscous solution to form a slurry;
spray dry granulating the slurry to form substantially homogeneous particles wherein the excipient comprises
about 75% to about 98% microcrystalline cellulose;
about 1% to about 10% at least one binder; and
about 1% to about 15% at least one disintegrant and wherein the binder is hydroxypropyl methylcellulose and the disintegrant is cross-linked polyvinylpyrrolidone.

8. The method of claim 7 comprising:
about 80% to about 90% microcrystalline cellulose;
about 2% to about 8% at least one binder; and
about 3% to about 12% at least one disintegrant.

9. The method of Claim 7 comprising:
about 85% to about 93% microcrystalline cellulose;
about 2% to about 5% at least one binder; and
about 10% at least one disintegrant.

10. A pharmaceutical tablet comprising:
at least one active pharmaceutical ingredient; and
an excipient of substantially homogeneous particles including:
a) microcrystalline cellulose;
b) at least one binder; and
c) at least one disintegrant
wherein the binder is hydroxypropyl methylcellulose and the disintegrant is cross-linked polyvinylpyrrolidone,
wherein the excipient is formed by spraying an aqueous slurry comprised of the microcrystalline cellulose, binder and disintegrant and
wherein the excipient includes:
75% to 98% microcrystalline cellulose;
1% to 10% said at least one binder; and
1% to 15% said at least one disintegrant.

11. The tablet of claim 10 wherein the excipient includes:
about 80% to about 90% microcrystalline cellulose;
about 2% to about 8% at least one binder; and
about 3% to about 12% at least one disintegrant.

12. The tablet of Claim 10 wherein the excipient includes:
about 85% to about 93% microcrystalline cellulose;
about 2% to about 5% at least one binder; and
about 10% at least one disintegrant.

13. A method of making a pharmaceutical tablet comprising: mixing at least one active pharmaceutical ingredient with a composition of substantially homogeneous particles according to claim 1 and compressing the mixture to form a tablet.

## Patentansprüche

1. Zusammensetzung, die im Wesentlichen homogene Partikel umfasst, die Folgendes beinhaltet:
etwa 75 % bis etwa 98 % mikrokristalline Cellulose;
etwa 1 % bis etwa 10 % wenigstens ein Bindemittel; und
etwa 1 % bis etwa 15 % wenigstens ein Sprengmittel,
wobei die mikrokristalline Cellulose, das Bindemittel und das Sprengmittel, wenn sie mit einem REM betrachtet werden, nicht unterscheidbar sind, wobei dadurch im Wesentlichen homogene kugelförmige Partikel ausgebildet werden, wobei das Bindemittel Hydroxypropylmethylcellulose ist und das Sprengmittel quervernetztes Polyvinylpyrrolidon ist,
wobei die Zusammensetzung durch Sprühen einer wässrigen Aufschlämmung ausgebildet wird, die aus der mikrokristallinen Cellulose, dem Bindemittel und dem Sprengmittel besteht.

2. Zusammensetzung nach Anspruch 1, wobei die Zusammensetzung Folgendes beinhaltet:
etwa 80 % bis etwa 90 % mikrokristalline Cellulose;
etwa 2 % bis etwa 8 % wenigstens ein Bindemittel; und
etwa 3 % bis etwa 12 % wenigstens ein Sprengmittel.

3. Zusammensetzung nach Anspruch 1, wobei die Zusammensetzung Folgendes beinhaltet:
etwa 85 % bis etwa 93 % mikrokristalline Cellulose;
etwa 2 % bis etwa 5 % wenigstens ein Bindemittel; und
etwa 10 % wenigstens ein Sprengmittel.

4. Verfahren zum Herstellen eines Arzneistoffträgers, das Folgendes umfasst:
Mischen eines Bindemittels in Wasser, um eine viskose Lösung auszubilden; Homogenisieren von mikrokristalliner Cellulose und einem Sprengmittel in die viskose Lösung, um eine Aufschlämmung auszubilden; und Sprühtrockengranulieren der Aufschlämmung, um im Wesentlichen homogene Partikel des Arzneistoffträgers auszubilden, wobei das Bindemittel Hydroxypropylmethylcellulose ist und das Sprengmittel quervernetztes Polyvinylpyrrolidon ist und wobei der Arzneistoffträger Folgendes umfasst:
75 % bis 98 % mikrokristalline Cellulose;
1% bis 10 % das wenigstens eine Bindemittel; und
1 % bis 15 % das wenigstens eine Sprengmittel.

5. Verfahren nach Anspruch 4, das Folgendes umfasst:
etwa 80 % bis etwa 90 % mikrokristalline Cellulose;
etwa 2 % bis etwa 8 % wenigstens ein Bindemittel; und
etwa 3 % bis etwa 12 % wenigstens ein Sprengmittel.

6. Verfahren nach Anspruch 4, das Folgendes umfasst:
etwa 85 % bis etwa 93 % mikrokristalline Cellulose;
etwa 2 % bis etwa 5 % wenigstens ein Bindemittel; und
etwa 10 % wenigstens ein Sprengmittel.

7. Verfahren zum Herstellen eines Arzneistoffträgers, das Folgendes umfasst:
Lösen von Bindemittel aus Hydroxypropylmethylcellulose in Wasser, um eine viskose Lösung auszubilden; Homogenisieren von mikrokristalliner Cellulose und einem Sprengmittel aus quervernetztem Polyvinylpyrrolidon in die viskose Lösung, um eine Aufschlämmung auszubilden;
Sprühtrockengranulieren der Aufschlämmung, um im Wesentlichen homogene Partikel auszubilden, wobei der Arzneistoffträger Folgendes umfasst:
etwa 75 % bis etwa 98 % mikrokristalline Cellulose;
etwa 1 % bis etwa 10 % wenigstens ein Bindemittel; und
etwa 1 % bis etwa 15 % wenigstens ein Sprengmittel und
wobei das Bindemittel Hydroxypropylmethylcellulose ist und das Sprengmittel quervernetztes Polyvinylpyrrolidon ist.

8. Verfahren nach Anspruch 7, das Folgendes umfasst:
etwa 80 % bis etwa 90 % mikrokristalline Cellulose;
etwa 2 % bis etwa 8 % wenigstens ein Bindemittel; und
etwa 3 % bis etwa 12 % wenigstens ein Sprengmittel.

9. Verfahren nach Anspruch 7, das Folgendes umfasst:
etwa 85 % bis etwa 93 % mikrokristalline Cellulose;
etwa 2 % bis etwa 5 % wenigstens ein Bindemittel; und
etwa 10 % wenigstens ein Sprengmittel.

10. Pharmazeutische Tablette, die Folgendes umfasst:
wenigstens einen pharmazeutischen Wirkstoff; und
einen Arzneistoffträger aus im Wesentlichen homogenen Partikeln, der Folgendes beinhaltet:
a) mikrokristalline Cellulose;
b) wenigstens ein Bindemittel; und
c) wenigstens ein Sprengmittel
wobei das Bindemittel Hydroxypropylmethylcellulose ist und das Sprengmittel quervernetztes Polyvinylpyrrolidon ist,
wobei der Arzneistoffträger durch Sprühen einer wässrigen Aufschlämmung ausgebildet wird, die aus der mikrokristallinen Cellulose, dem Bindemittel und dem Sprengmittel besteht und wobei der Arzneistoffträger Folgendes beinhaltet:
75 % bis 98 % mikrokristalline Cellulose;
1% bis 10 % das wenigstens eine Bindemittel; und
1 % bis 15 % das wenigstens eine Sprengmittel.

11. Tablette nach Anspruch 10, wobei der Arzneistoffträger Folgendes beinhaltet:
etwa 80 % bis etwa 90 % mikrokristalline Cellulose;
etwa 2 % bis etwa 8 % wenigstens ein Bindemittel; und
etwa 3 % bis etwa 12 % wenigstens ein Sprengmittel.

12. Tablette nach Anspruch 10, wobei der Arzneistoffträger Folgendes beinhaltet:
etwa 85 % bis etwa 93 % mikrokristalline Cellulose;
etwa 2 % bis etwa 5 % wenigstens ein Bindemittel; und
etwa 10 % wenigstens ein Sprengmittel.

13. Verfahren zum Herstellen einer pharmazeutischen Tablette, das Folgendes umfasst:
Mischen wenigstens eines pharmazeutischen Wirkstoffs mit einer Zusammensetzung aus im Wesentlichen homogenen Partikeln nach Anspruch 1 und Komprimieren der Mischung, um eine Tablette auszubilden.

## Revendications

1. Composition comprenant des particules sensiblement homogènes comprenant :
environ 75% à environ 98% de cellulose microcristalline;
environ 1% à environ 10% d'au moins un liant; et
environ 1% à environ 15% d'au moins un désintégrant,
dans laquelle la cellulose microcristalline, le liant et le désintégrant sont indiscernables quand observés avec un MEB, formant ainsi des particules sphériques sensiblement homogènes, dans laquelle le liant est de l'hydroxypropylméthylcellulose et le désintégrant est de la polyvinylpyrrolidone réticulée,
dans laquelle la composition est formée par pulvérisation d'une suspension épaisse aqueuse constituée de la cellulose microcristalline, du liant et du désintégrant.

2. Composition selon la revendication 1, dans laquelle la composition comprend :
environ 80% à environ 90% de cellulose microcristalline;
environ 2% à environ 8% d'au moins un liant; et
environ 3% à environ 12% d'au moins un désintégrant.

3. Composition selon la revendication 1, dans laquelle la composition comprend :
environ 85% à environ 93% de cellulose microcristalline;
environ 2% à environ 5% d'au moins un liant; et
environ 10% d'au moins un désintégrant.

4. Procédé de fabrication d'un excipient comprenant les étapes consistant à :
mélanger un liant dans de l'eau pour former une solution visqueuse; homogénéiser de la cellulose microcristalline et un désintégrant dans la solution visqueuse pour former une suspension épaisse; et pulvériser à sec la granulation de la suspension épaisse pour former des particules sensiblement homogènes d'excipient dans lequel le liant est de l'hydroxypropylméthylcellulose et le désintégrant est de la polyvinylpyrrolidone réticulée et dans lequel l'excipient comprend
75% à 98% de cellulose microcristalline;
1% à 10% dudit au moins un liant; et
1% à 15% dudit au moins un désintégrant.

5. Procédé selon la revendication 4, comprenant :
environ 80% à environ 90% de cellulose microcristalline;
environ 2% à environ 8% d'au moins un liant; et
environ 3% à environ 12% d'au moins un désintégrant.

6. Procédé selon la revendication 4, comprenant :
environ 85% à environ 93% de cellulose microcristalline;
environ 2% à environ 5% d'au moins un liant; et
environ 10% d'au moins un désintégrant.

7. Procédé de fabrication d'un excipient comprenant les étapes consistant à :
dissoudre un liant d'hydroxypropylméthylcellulose dans de l'eau pour former une solution visqueuse;
homogénéiser de la cellulose microcristalline et de la polyvinylpyrrolidone réticulée dans la solution visqueuse pour former une suspension épaisse;
pulvériser à sec la granulation de la suspension pour former des particules sensiblement homogènes et dans lequel l'excipient comprend :
environ 75% à environ 98% de cellulose microcristalline;
environ 1% à environ 10% d'au moins un liant; et
environ 1% à environ 15% d'au moins un désintégrant et
dans lequel le liant est de l'hydroxypropylméthylcellulose et le désintégrant est de la polyvinylpyrrolidone réticulée.

8. Procédé selon la revendication 7, comprenant:
environ 80% à environ 90% de cellulose microcristalline;
environ 2% à environ 8% d'au moins un liant; et
environ 3% à environ 12% d'au moins un désintégrant.

9. Procédé selon la revendication 7, comprenant:
environ 85% à environ 93% de cellulose microcristalline;
environ 2% à environ 5% d'au moins un liant; et
environ 10% d'au moins un désintégrant.

10. Comprimé pharmaceutique comprenant:
au moins un ingrédient pharmaceutique actif; et
un excipient de particules sensiblement homogènes comprenant:
a) de la cellulose microcristalline;
b) au moins un liant; et
c) au moins un désintégrant,
dans lequel le liant est de l'hydroxypropylméthylcellulose et le désintégrant est de la polyvinylpyrrolidone réticulée, dans lequel l'excipient est formé en pulvérisant une suspension épaisse aqueuse comprenant la cellulose microcristalline, le liant et le désintégrant et
dans lequel l'excipient comprend:
75% à 98% de cellulose microcristalline;
1% à 10% dudit au moins un liant; et
1% à 15% dudit au moins un désintégrant.

11. Comprimé selon la revendication 10, dans lequel l'excipient comprend:
environ 80% à environ 90% de cellulose microcristalline;
environ 2% à environ 8% d'au moins un liant; et
environ 3% à environ 12% d'au moins un désintégrant.

12. Comprimé selon la revendication 10, dans lequel l'excipient comprend:
environ 85% à environ 93% de cellulose microcristalline;
environ 2% à environ 5% d'au moins un liant; et
environ 10% d'au moins un désintégrant.

13. Procédé de fabrication d'un comprimé pharmaceutique comprenant les étapes consistant à: mélanger au moins un ingrédient pharmaceutique actif avec une composition de particules sensiblement homogènes selon la revendication 1 et compresser le mélange pour former un comprimé.
